# EUROPEAN PATENT APPLICATION

(11) **EP 2 052 727 A2**
(43) Date of publication of application: **29.04.2009**
(21) Application number: 07117735.6
(22) Date of filing: 02.10.2007
(51) Int. Cl.: A61K 36/27, A61P 3/04

(54) **Hoodia extract oil compositions comprising unsaturated monoacylglycerides**

(71) Applicant: Unilever N.V., 3013 AL Rotterdam (NL)
(72) Inventor: The designation of the inventor has not yet been filed
(74) Representative: Wurfbain, Gilles L.

(57) **Abstract**

Oil compositions containing Hoodia plant extract dissolved in an unsaturated mono-acyl-glyceride. Edible emulsions comprising the inventive oil compositions are also disclosed.

## Description

### TECHNICAL FIELD

The present invention relates generally to the field of appetite suppressant compositions. More particularly, it relates to appetite suppressant compositions comprising oil compositions including plant extracts of steroidal glycosides, especially extracts from the plants of the *Hoodia* family.

### BACKGROUND OF THE INVENTION

Extracts obtainable from plants of the *Apocynaceae* family (also known as the *Asclepiadaceae* family), particularly the *Hoodia* genus (formerly the *Hoodia* and *Trichocaulon* genera) have been shown to have an appetite suppressant activity and are potentially useful in weight management products. US Patent 6,376,657 discloses that these plants contain steroidal glycosides. US Patent 6,376,657 also discloses processes to extract steroidal glycosides from *Hoodia* plants.

US 2005/0202103 (Rajendran et al.) discloses steroidal glycosides obtainable from Caralluma, another genus of plants in *Asclepidaceae* family. US 7,008,648 (Corley et al.) discloses steroidal glycosides obtainable from *Stapelia* and *Orbea* plants (also within the *Asclepidaceae* family). WO 2005/099737 discloses additional steroidal glycosides obtainable from *Asclepiadaceae* plants.

Compositions comprising phytosterols or derivatives dissolved or dispersed in fats and oils are described in US 6,139,897, US 2002/0045000, US 6,326,050, US 2004/0037940, US 2006/0062888 (Kao Corporation). WO 03/055324 (Raisio Benecol) discloses compositions comprising emulsified plant sterols.

WO 2004/071399 (Phytopharm) discloses pharmaceutical compositions comprising *Hoodia* extract and various oils as diluents. WO 2006/069619 (Unilever) discloses edible compositions which may include *Hoodia* extract in a co-lipid composition of the first lipid which is a reaction product of one or more carboxylic acids with propylene glycol or glycerol and the second lipid which may be a hydroxylated carboxylic acid ester of mono- and di-acyl-glycerides.

The present is based at least in part on the discovery that it is difficult to formulate suitable food products comprising *Hoodia* extracts, because the extracts and the pure compounds are solid at room temperature and have very high melting temperature (e.g., Hoodia extract with 70% actives has a melting point above 200°C). Moreover, they are not soluble in water and poorly soluble in commonly used edible fats--saturated or unsaturated long chain tri-acyl-glycerides.

The poor solubility of the Hoodia plant extract in common edible (food grade) oils or water leads to the formation of large solid aggregates, which impart physical instability resulting in a gritty texture, enhanced sedimentation poor palatability and unacceptable appearance of the foods. Furthermore, the poor dispersibility of the material makes processing and cleaning of the food processing equipment difficult and can lead to problems when the material is mixed with or dispersed in other food ingredients (e.g., physical instability).

Higher solubility in an oil is desirable as it would lead to reduced minimum required oil content in final compositions (which is desirable for weight management products), the ability to prepare concentrated solutions of the plant extracts in oil, improved stability, appearance, organoleptic properties and improved bioavailability. Higher solubility also insures that preferred steroidal glycosides, out of those that are initially present in the extract, are present at the same or nearly the same concentrations in the final solution.

Finally, even when acceptable food products can be produced, the bioavailability of the active compounds may not be optimal - the bioavailability is affected, in part, by being able to provide an isotropic mixture of an oil, emulsifier and Hoodia, which form oil-in-water emulsions upon aqueous dilution under conditions in the gastrointestinal tract.

Accordingly, there is a need for commercially acceptable edible appetite suppressant emulsions comprising *Hoodia* plant extracts.

The present invention is based at least in part on the unexpected discovery that *Hoodia* plant extracts containing steroidal glycosides can be solubilised in certain food-grade oils.

### SUMMARY OF THE INVENTION

In a first aspect the invention relates to oil compositions comprising:
(i) from about 10% to about 95%, by weight of the composition, of a food grade mono-acyl-glyceride of an unsaturated fatty acid;
(ii) from about 1 % to about 90%, by weight of the composition, of Hoodia plant extract comprising steroidal glycosides.

In a second aspect, the invention relates to emulsions comprising the inventive compositions.

### DETAILED DESCRIPTION OF THE INVENTION

Except in the examples, or where otherwise explicitly indicated, all numbers in this description indicating amounts of material or conditions of reaction, physical properties of materials and/or use are to be understood as modified by the word "about." All amounts are by weight, unless otherwise specified.

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

"Dissolved" as used herein means forming a single phase when a mixture is visibly examined.

"Food-grade" as used herein means non-toxic edible composition.

"Steroidal glycoside" as used herein means a steroid (four fused rings in a 6-6-6-5 pattern), further comprising at least one side group substitution which is a glycoside (a molecule in which a sugar group is bonded through its anomeric carbon to another group via an O-glycosidic bond), preferably a deoxy or dideoxy glycoside, and another side group comprising a carbonyl (-C=O) group, preferably located on the 5-membered ring and most preferably further comprising a tigloyl group 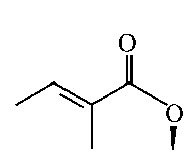 on carbon number 12.

### MONOACYLGLYCERIDES OF UNSATURATED OR POLYUNSATURATED FATTY ACIDS ("UMAG")

According to the present invention, by virtue of including an oil of certain composition, specifically a UMAG, Hoodia plant extract containing a relatively high amount of steroidal glycosides may be dissolved. By virtue of employing the unsaturated mono-acyl-glycerides, the processing becomes commercially feasible, since UMAG has low melting point and so is liquid at ambient temperature; and the health benefit of the inventive composition is increased.

UMAG is obtained as either: a condensation reaction product of one unsaturated or polyunsaturated fatty acid with glycerol, or a product of cleavage of one fatty acid from a di-acyl-glyceride molecule, or a product of cleavage of two fatty acids from a tri-acyl-glyceride molecule.

Preferred mono- or polyunsaturated fatty acids include at least 12 carbon atoms, most preferably at least 14 carbon atoms. Examples include but are not limited to:

| | | |
|---|---|---|
| Myristoleic acid: | CH₃(CH₂)₃CH=CH(CH₂)₇COOH | C14:1 |
| Palmitoleic acid: | CH₃(CH₂)₅CH=CH(CH₂)₇COOH | C16:1 |
| Oleic acid: | CH₃(CH₂)₇CH=CH(CH₂)₇COOH or *cis*-Δ⁹ | C18:1 |
| Linoleic acid: | CH₃(CH₂)₄CH=CHCH₂CH=CH(CH₂)₇COOH | C18:2 |
| Alpha-linolenic acid: | CH₃CH₂CH=CHCH₂CH=CHCH₂CH=CH(CH₂)₇COOH | C18:3 |
| Arachidonic acid | CH₃(CH₂)₄CH=CHCH₂CH=CHCH₂CH=CHCH₂CH=CH (CH₂)₃COOH | C20:4 |
| Eicosapentaenoic acid | CH₃(CH₂)CH=CHCH₂CH=CHCH₂CH=CHCH₂CH=CHC H₂CH=CH(CH₂)₃COOH | C20:5 |
| Erucic acid: | CH₃(CH₂)₇CH=CH(CH₂)₁₁COOH | C22:1 |
| Docosahexaenoic acid | CH₃(CH₂)CH=CHCH₂CH=CHCH₂CH=CHCH₂CH=CHC H₂CH=CHCH₂CH=CH(CH₂)₂COOH | C22:6 |

The above also represents the description of fatty acid residues in the UMAG. Major tri-acyl-glyceride oils which may be used to produce UMAG or which may be included as further optional ingredient in inventive composition:
Coconut oil, Corn oil, Cottonseed oil, Canola oil, rapeseed oil, Olive oil, Palm oil, Peanut oil, Safflower oil, Sesame oil, Soybean oil, Sunflower oil, Almond oil, Cashew oil, Hazelnut oil, Macadamia oil, Pecan oil, Pistachio oil, Walnut oil, Acai oil, grape seed oil, Blackcurrant seed oil, Borage seed oil, Evening primrose oil, Amaranth oil, Apricot oil, Argan oil, Avocado oil, Babassu oil, Ben Carob pod oil, Coriander seed oil, False flax oil, Hemp oil, Kapok seed oil, Meadowfoam seed oil, Mustard oil (pressed), Okra seed oil (Hibiscus seed oil), Perilla seed oil, Pine seed oil, Poppyseed oil, Prune kernel oil, Pumpkin seed oil, Quinoa oil, Rice bran oil, Tea oil (Camellia oil), Thistle oil, Wheat germ oil, Illipe oil, Cohune oil, Premier Jus oil,Tallow, Lard, Butter, Ghee, Fish oil (Cod, Herring, Menhaden, Pilchard, Sardine, etc).

UMAG includes derivative of UMAG including, but not limited to, acetylated-UMAG, citrylated-UMAG and lactylated-UMAG.

UMAG is included in the present invention in an amount of from 10% to 95%, preferably from 20% to 90%, most preferably from 40% to 90%, and optimally from 70% to 90% by weight of the oil composition.

### EMULSIFIER

Suitable emulsifiers are selected surface active agents from the group of proteins (e.g., milk, soy, casein, whey), surfactants (see e.g., Industrial Surfactants (2nd Edition) By: Flick, Ernest W. 1993 William Andrew Publishing/Noyes), and (bio) polymers (e.g., OSA starch, gum arabic).

According to the preferred embodiment of the invention, certain emulsifiers are combined with UMAG to enhance Hoodia solubility, to enhance compatibility with gastro-intestinal media and to facilitate the preparation of emulsions.
Particularly preferred emulsifiers are selected from the group consisting of propylene glycol carboxylic acid mono-ester, polyglyceryl oleate and diethylene glycol monoethyl ether, in order to obtain better miscibility with UMAG and better miscibility of the resulting mixture with gastrointestinal media; most preferably the emulsifiers are propylene glycol monolaurate, propylene glycol monocaprylate and polyglyceryl oleate. Especially preferred, to obtain optimum solubility and miscibility with gastrointestinal media, are propylene glycol monocaprylate and propylene glycol monolaurate.

When these emulsifiers are included, it is present in an amount of from 0.1 % to 85%, preferably from 5% to 70%, most preferably from 10% to 60%, and optimally from 15% to 50%, by weight of the oil composition.

The emulsifier and the UMAG are employed in the ratio of from 5:95 to 95:5, preferably (to improve stability and miscibility of oil phase with intestinal or gastrointestinal media) from 20:80 to 80:20, most preferably from 30:70 to 70:30 and optimally from 40:60 to 60:40.

### Hoodia Plant Extracts

The steroidal glycoside in the present invention is delivered to inventive compositions from extracts of plants of the *Hoodia* (also known as *Trichocaulon)* genus.

More preferably, the plant extract is selected from the group consisting of *Trichocaulon piliferum* extracts, *Trichocaulon officinale* extracts, *Hoodia currorii* extracts, *Hoodia gordonii* extracts, *Hoodia lugardii* extracts and mixtures thereof. *Hoodia gordonii* extracts are the most preferred due to the clinically proven safety of use and efficacy in appetite suppression.

The plant extracts are preferably of certain minimum purity in order to attain the best solubility in an oil. According to the present invention the extract preferably comprises at least 15%, by weight of the extract, of steroidal glycosides, preferably at least 30%, more preferably at least 50%, most preferably at least 70% or better at least 80% and optimally at least 90%. US Patent 6,376,657, incorporated by reference herein, describes the preparation of a suitable extract comprising steroidal glycosides from the genus *Hoodia,* said steroidal glycoside having appetite suppressant activity. The solvents specifically mentioned to perform the extraction are one or more of methylene chloride (dichloromethane), water, methanol, hexane, ethyl acetate or mixtures thereof. An alternative method to obtain an extract is disclosed by separating plant sap from the plant solid material. Other methods of extracting a steroidal glycoside from plants are also suitable.

"Extract" as used herein includes solvent-extracted liquid, solid or spray-dried or freeze-dried forms of extracts, sap, which also may be purified, partially purified, concentrated and/or fractionated, or otherwise concentrated plant preparation. Solvent extracted forms of the extract are preferred due to higher purity and process efficacy.

Suitable steroidal glycoside compounds include but are not limited to the general structural formula (1): 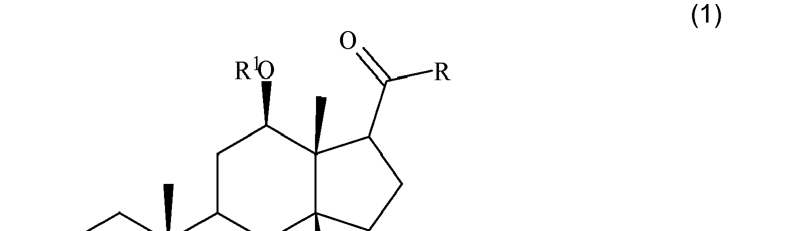 wherein
R = alkyl;
R¹ = H, alkyl, tigloyl, benzoyl or any other organic ester group, most preferably to optimize efficacy tigloyl;
R² = H or one or more 6-deoxy carbohydrates, or one or more 2,6-dideoxy carbohydrates, or glucose radical, or combinations thereof; and wherein the broken lines indicate the optional presence of a further bond between carbon atoms C4 and C5 or between carbon atoms C5 and C6.

Particularly preferred steroidal glycosides are analogs of Compound of Formula 1, including Compounds of Formula (2) through Formula (8), and mixtures thereof, since these are obtainable from the preferred *Hoodia* plants.

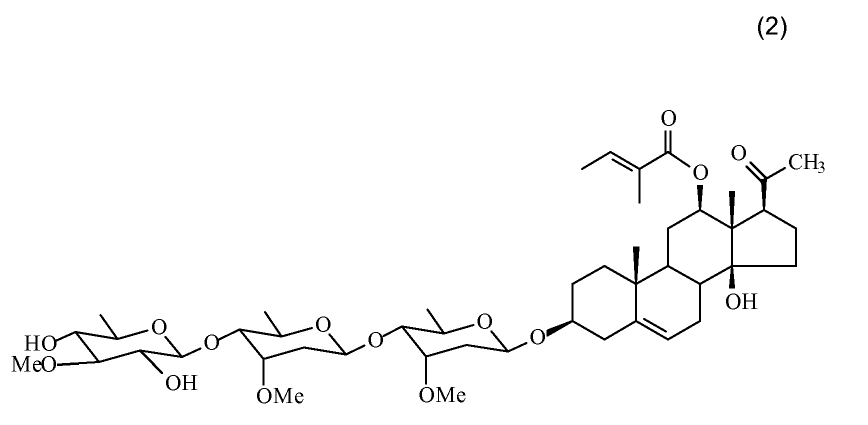

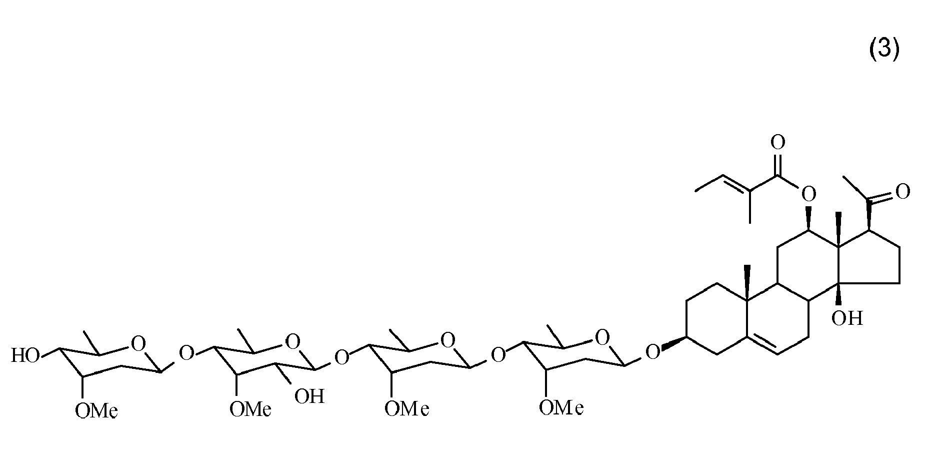

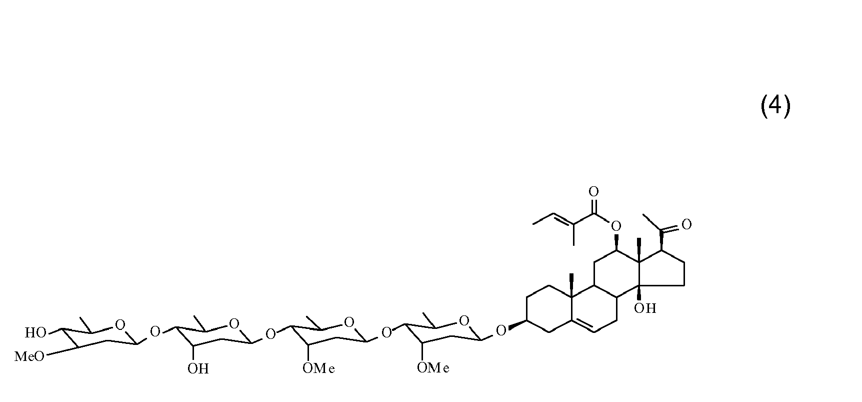

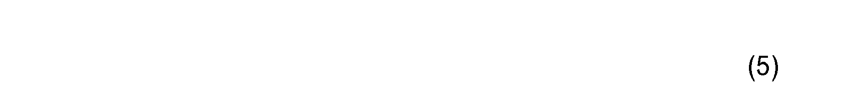

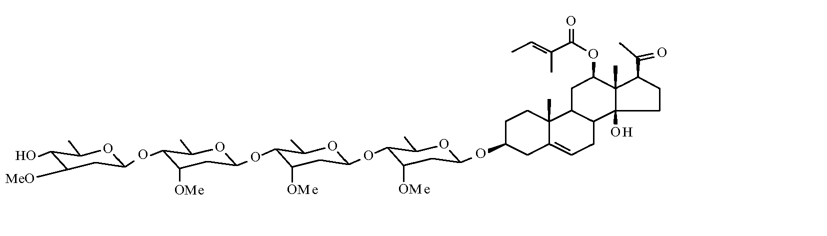

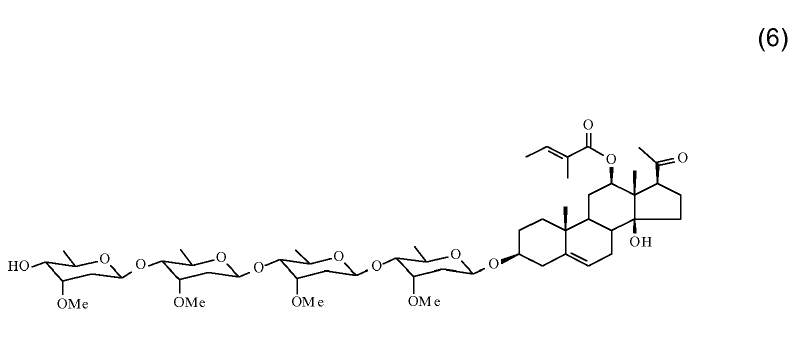

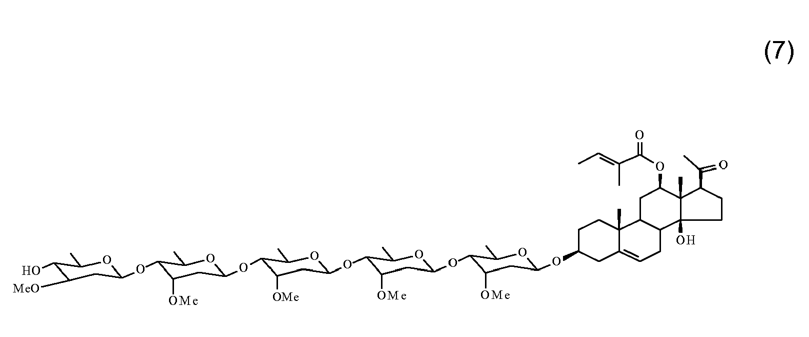

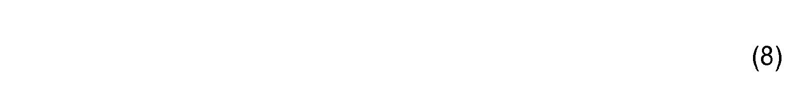

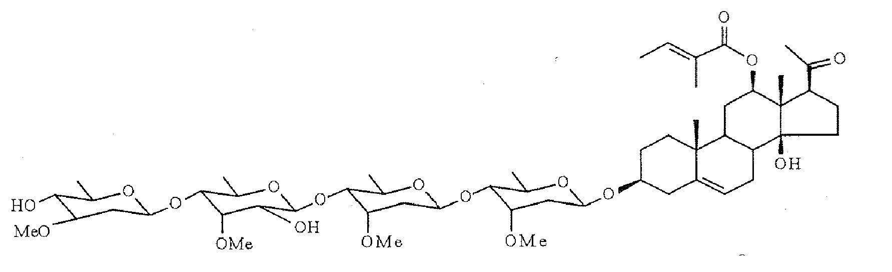

Other steroidal glycosides not specifically mentioned herein may be included in the inventive product. It will be understood that the invention also encompasses isomers, derivatives, salts, esters and analogs of the steroidal glycosides (preferably, biologically active) and mixtures thereof.
Steroidal glycoside concentrations are determined using high performance liquid chromatography (HPLC) with UV detection after extraction or dissolution.

Approximately 20 mg of the material is dissolved in 50 ml of methanol by sonication for 10 minutes. After filtration 1 ml of the filtrate is evaporated to dryness and reconstituted in 1 ml acetonitrile/water (50/50 v/v).

Steroidal glycosides are measured by LC-UV at 220 nm. To this end 20 µl of the extracts are injected onto a Zorbax RX-C8 analytical column of 250 x 4.6 mm packed with 5 µm particles and equipped with a Zorbax RX-C8 guard column of 12.5 x 4.6 mm packed with the same stationary phase. The column system is held at 40°C. Gradient elution is performed starting at 41.2% acetonitrile/methanol (85/15 v/v) and 58.8% water/methanol (85/15 v/v) at a flow rate of 1 ml/min. Initial conditions are held for 10 minutes before being linearly increased to 88.2% acetonitrile/methanol (85/15 v/v) and 11.8% water/methanol (85/15 v/v) over 30 minutes. After a final hold of 5 minutes the system is re-equilibrated to the starting conditions. For the oil samples an adapted gradient was used (see Table 1).

**Table 1: HPLC gradient for the analysis of Hoodia steroidal glycosides in oil.**

| Gradient | Time(min) % water | | %acetonitrile % | methanol | Flow(ml/min) |
|---|---|---|---|---|---|
| *10 min isocratic hold* | 0 | 50 | 35 | 15 | 1.0 |
| *30 min linear gradient* | 10 | 50 | 35 | 15 | 1.0 |
| *5 min isocratic hold* | 40 | 10 | 75 | 15 | 1.0 |
| | 45 | 10 | 75 | 15 | 1.0 |
| | 50 | 50 | 35 | 15 | 1.0 |
| Flush | 55 | 1 | 1 | 98 | 2.0 |
| | 85 | 1 | 1 | 98 | 2.0 |
| *Reconditioning* | 90 | 50 | 35 | 15 | 1.0 |
| | 120 | 50 | 35 | 15 | 1.0 |

Compound of Formula 2 of any known purity (95% was used in this case) is used for calibration. Compound 2 may be isolated from an extract of dried *Hoodia* gordonii using preparative liquid chromatography or may be synthesized (see e.g. US Patent 6,376,657, incorporated by reference herein). A stock solution at 100 µg/ml is prepared in acetonitrile/water (1/1 v/v) and further dilutions are prepared to yield additional calibration standards at 75, 50, 20, 10 and 5 µg/ml. UV response at 220 nm is used for quantification against the Compound 2 calibration line. Relative response factors based on molecular weight are used to quantify the steroidal glycosides against the Compound 2 calibration line. Steroidal glycosides are defined as all peaks eluting after 15 min that are not present in the blank acetonitrile/water (1/1 v/v) sample. For the compounds of Formula 2 - 8 the specific relative retention times and response factors, are summarized in Table 2.

**Table 2: Relative retention times and response factors of some steroidal glycosides**

| Compound | Relative retention time vs. Compound 2 | Response factor vs. Compound 2 |
|---|---|---|
| formula 2 | 1.000 | 1.000 |
| formula 8 | 1.066 | 1.164 |
| formula 3 | 1.128 | 1.164 |
| formula 4 | 1.191 | 1.130 |
| formula 5 | 1.292 | 1.146 |
| formula 6 | 1.328 | 1.146 |
| formula 7 | 1.399 | 1.309 |

The other steroidal glycosides peaks eluting after 15 minutes have a response factor of 1.081 vs. Compound 2.

The inventive compositions include from 1% to 90% of Hoodia extract, more preferably from 4% to 70%, most preferably from 7% to 60%, optimally from 10% to 50%.

It is most preferred to provide Hoodia oil compositions which contain not just Hoodia extract dissolved in oil, but a fully isotropic mixture of Hoodia extract, a food-grade oil and a food-grade emulsifier.

The preferred inventive oil compositions have a green color. When incorporated into the preferred oil-in-water emulsions, these oil compositions may then be present as green droplets, which may be useful to signal to the consumer the healthy, natural "green" composition and/or the presence of Hoodia extract.

The oil compositions according to the invention are preferably physically stable for at least 1 week, more preferably for 3 weeks, most preferably for 3 months, and optimally for at least 1 year at ambient temperature.

The oil compositions according to the invention preferably have the viscosity at ambient temperature in the range of from 0.0001 Pa.s to 10000 Pa.s, preferably from 0.001 Pa.s to 1000 Pa.s, most preferably from 0.001 Pa.s to 100 Pa.s in order to facilitate the delivery or the further processing of these compositions.

The preferred oil compositions are preferably miscible (self-emulsifying) with gastrointestinal media.

### EMULSIONS

In another aspect of the invention, the oil composition according to the invention is employed to produce the inventive emulsion compositions which include the oil phase comprising the oil composition as described above and the aqueous phase.

The oil-in-water emulsions according to the invention incorporate the oil phase (which is dispersed) in an amount of from to 0.1 % to 90%, preferably from 0.5% to 30%, most preferably from 0.8 %to 20%, and optimally from 1.0 % to 10%.

The water-in-oil emulsions according to the invention incorporate the oil phase (which is continuous) in an amount of from 10% to 99.9%, preferably from 20% to 90%, most preferably from 30% to 70%, and optimally from 40% to 60%.

### METHOD OF MAKING

Preferably, the composition is prepared in two steps.
First extract from *Hoodia gordonii* is mixed with the oils phase under continuous stirring. To accelerate to process of dissolution, temperature can be elevated to 80-90C and/or ultrasound can be used.

In the second step an emulsion is prepared using methods and processing known in the art (e.g. see Encyclopedia of Emulsion Technology, D. Schuster, Marcel Dekker, 1983, New York). The resulting emulsion can be added to the rest of the products or the rest of ingredient can be added to the emulsion. Alternatively, the emulsion can be prepared in the presence of the other ingredients.

### OPTIONAL INGREDIENTS

The inventive composition (oil compositions and/or emulsions) preferably include proteins, additional fats/oils, and carbohydrates.

Suitable proteins include but are not limited to milk and milk derived proteins, egg and egg derived proteins, plant or vegetable and plant or vegetable derived proteins, soy and soy derived proteins, meat or fish and meat or fish derived proteins, cereal and cereal derived proteins, as well as combinations thereof. Examples include but are not limited to milk, skimmed milk, fat free milk, condensed milk, fermented milk, cream, whey, yoghurt, cheese, egg, buttermilk, milk powder, buttermilk powder, cream powder, whey powder, yoghurt powder, cheese powder, egg powder, calcium and sodium caseinates, lactose free dairy proteins, soy proteins, isolated soy proteins, vegetable proteins, meat and fish derived proteins, gelatin, albumin powder, and mixtures thereof.

In the most preferred embodiment of the invention, an optimum balance between the amount of proteins and steroidal glycoside amount is maintained, in the range of from 0.5:1 to 200:1 (mg protein: mg steroidal glycoside), preferably from 2:1 to 100:1, most preferably from 5:1 to 75:1.

Suitable carbohydrates include but are not limited to potato, pasta, wheat, corn, soy fiber, fruit fiber (e.g. apple and orange), sucrose, fructose, dextrose, lactose, maltodextrins, honey, corn syrup, oligofructose, starches (e.g. otato starch, corn starch, rice starch), modified starches, fruit juice, concentrated fruit juice, flours (e.g. wheat, corn and rice), gums (e.g. xanthan gum, guar gum, gum arabic, locust bean gum, celluloses and modified celluloses (e.g. odium carboxy methyl cellulose, microcrystalline cellulose, powdered cellulose), carageenan, potassium carageenan, alginates (e.g. odium and potassium alginates), gelatin, pectin and mixtures thereof.

It should be noted that some or all of the sugar may be replaced by artificial sweeteners, or artificial sweeteners may be present in addition to sugars. Artificial sweeteners include but are not limited to aspartame, cyclamates (e.g. Sodium cyclamate), acesulfame K, sucralose, saccharin, invert sugar, maltose sugar, sugar alcohols (e.g. maltitol, sorbitol) and mixtures thereof.
Suitable fats include but are not limited to saturated and unsaturated fats and oils, for instance sunflower oil, high oleic sunflower oil, canola, cottonseed oil, corn/maize oil, rapeseed oil, olive oil, soybean oil, palm oil, palm kernel oil, coconut, fish oil, linseed oil, peanut/groundnut oil, safflower oil, sesame oil, butter, lard, cocoa butter, mono- and di-acyl-glycerides, which may include saturated acyl glycerides, and mixtures thereof.

The sources of oils and fats can also be hardened (e.g. by hydrogenation) or fractionated (e.g. via solvents) and these can be mixed with other oils or fats. In order to optimise the health value of the inventive products, at least some of the fat, generally from 10 to 80%, preferably from 30 to 50%, by weight of the total fat, is present as unsaturated or polyunsaturated oil, in particular oils which contain linoleic (e.g. unflower, soybean, corn, Linola^{tm} or rapeseed) or linolenic acid (e.g. linseed) and mixtures thereof. Ideally the product should deliver at least 1g per day of linoleic and/or linolenic acid, preferably from an unsaturated fat source.

To minimise the potential harmful effects, the inventive products are substantially free of trans fat, i.e. contain less then 0.5% of trans fat, preferably less then 0.1 %, most preferably less than 0.05% and optimally 0% trans fat, by weight of the product.

The preferred inventive compositions include essential minerals selected from the group consisting of phosphorus, iron, zinc, copper, selenium, magnesium, manganese, molybdenum and chromium, especially iron, phosphorous and zinc, and most especially iron as these are considered essential for the nutrition and the lack of one or more of these, and especially iron, in sufficient amounts may lead to health problems. The minerals described in this paragraph are added minerals rather than the trace amounts that may be present in various raw ingredients.

The preferred ingredients to deliver these minerals include but are not limited to magnesium oxide, ferrous sulfate, ferrous lactate, ferrous fumarate, ferric pyrophosphate, ferric orthophosphate, carbonyl iron, electrolytic iron, NaFeEDTA, zinc oxide, zinc gluconate, chromium chloride, sodium selenate, manganese sulfate and mixtures thereof.

The various minerals are included in the inventive products in the amounts as follows:

| | Units | Range (per unit serving) | | Preferred range(per unit serving) | |
|---|---|---|---|---|---|
| Mineral | | Low | High | Low | High |
| Phosphorous | Mg | 100 | 1000 | 300 | 500 |
| Iron | Mg | 1 | 10 | 3 | 7 |
| Zinc | Mg | 1 | 7 | 2 | 6 |
| Magnesium | µg | 20 | 200 | 80 | 150 |
| Selenium | µg | 3 | 40 | 7 | 25 |
| Chromium | µg | 5 | 50 | 10 | 42 |
| Molybdenum | µg | 3 | 40 | 7.5 | 27 |
| Manganese | Mg | 0.05 | 6 | 0.2 | 1 |
| Copper | Mg | 0.1 | 2 | 0.2 | 0.6 |

In particular the range of steroidal glycosides to iron is from 25:1 to 500:1 (mg steroidal glycosides: mg iron), preferably from 40:1 to 400:1, most preferably 50:1 to 300:1.

The inventive composition preferably further includes additional nutrients, vitamins and additional minerals to deliver healthy nutrition, despite the appetite suppression. Suitable vitamins and minerals, include but are not limited to Vitamin A, Vitamin D, Vitamin E, Vitamin C, Thiamin, Riboflavin, Niacin, Vitamin B6, folate, Vitamin B12, Biotin, Pantothenic acid, Calcium, Potassium, Sodium, iodine, vitamin K, and mixtures thereof.

The preferred ingredients to deliver vitamins and minerals include but are not limited to potassium phosphate, calcium phosphate, magnesium oxide, magnesium phosphate, ascorbic acid, sodium ascorbate, vitamin E acetate, niacinamide, ferric orthophosphate, calcium pantothenate, zinc oxide, zinc gluconate, vitamin A palmitate, pyridoxine hydrochloride, riboflavin, thiamin mononitrate, biotin, folic acid, chromium chloride, potassium iodide, sodium molybdate, sodium selenate, phytomenadione (vitamin K), cholecalciferol (vitamin D3), cyanocobalamin (vitamin B12), manganese sulfate and mixtures thereof. Preferably, the inventive product contains at least 10% or more of the recommended daily amount ("RDA") of the vitamins and minerals.

Another especially preferred optional ingredient is fiber. Suitable fiber sources include but are not limited to: inulin /oligofructose, gum arabic, cellulose, cellulose gum, wheat fiber (nutriose), fruit pulp/fiber, pectin, guar gum. Fiber is included generally in an amount of from 0.5-10 g per product, preferably 0.8 to 8, most preferably from 1 to 5.

The inventive products may further include meat, fish, meat and fish extracts, fruit, dried fruit, fruit concentrates, fruit extracts, fruit juices, tea (e.g. green tea) vegetables, vegetable extracts and concentrates, nuts, nut extracts, chocolate, bread, vinegar, salt, pepper, cocoa powder, herbs (e.g. parsley), herb extracts, spices (e.g. cinnamon), spice extracts, emulsifiers, acidity regulators (e.g. phosphoric, malic, maleic, citric, tartaric acids and salts thereof), flavonoids, preservatives (e.g. lactic acid, EDTA, tocopherols, sodium benzoate), colors (e.g. beta carotene, lycopene, caramel, carmine red), fibers (e.g. soy), leavening agents (e.g., sodium bicarbonate), pectin, citric acid, yeast, salt, glycerine, and mixtures thereof.
The preferred inventive products are substantially free of cholesterol, i.e. the products comprise less than 10 mg of cholesterol, preferably no more than 5 mg per unit serving, and optimally are free of cholesterol. The preferred products include phyto-sterols and/or phyto-stanols for cholesterol lowering effects.

The preferred inventive products comprise less than 6 g of sodium, preferably less than 3g, most preferably less than 1g, optimally less than 150 mg per unit serving.

The preferred inventive products contain at least 70 mg of potassium, preferably at least 100 mg, most preferably at least 140 mg per unit serving.

### PRODUCTS

The final products according to the invention may be in solid or liquid form. The particular advantage of the invention is in delivering liquid products although numerous advantages exist in processing the liquid components of the solid products in the plant and on storage. The liquid products according to the invention are preferably oil-in water emulsions wherein the steroidal glycoside containing plant extracts are dissolved in an oil phase.

The preferred product format of the edible appetite suppressant product is a unit serving drink or bar. A bar weight is from 20 to 100 g, preferably from 25 to 75 g, most preferably from 25 to 60 g. A drink has a volume of 80 to 600 ml, preferably 90 to 400 ml, most preferably from 100 to 350 ml. Preferably each unit serving is separately packaged and includes instructions, in particular recommendation of the number of the particular unit serving to be consumed per day.

Unit serving products of the invention contain from 50 to 2000 mg steroidal glycosides, preferably from 70 to 1500 mg, more preferably from 80 to 1200 mg, and optimally from 100 to 1000 mg.

The especially preferred unit serving drink according to the invention has the following composition, in addition to the steroidal glycoside:
75-95 wt% (preferably 80-90 wt%) moisture
0.5-10 wt% (preferably 1-7 wt%) protein
0.5-6 wt% (preferably 0.6-5 wt%) fat (including the oils of the inventive oil composition)
3-20 wt% (preferably 4-15 wt%) carbohydrate and
up to 8 wt% (preferably 1-6 wt%) minor components

The especially preferred unit serving bar according to the invention has the following composition, in addition to the steroidal glycoside:
3-30 wt% (preferably 5-25 wt%) moisture
3-30 wt% (preferably 5-25 wt%) protein
3-30 wt% (preferably 5-25 wt%) fat (including the oils of the inventive oil composition)
35-80 wt% (preferably 40-75 wt%) carbohydrate and
up to 12 wt% (preferably 1-10 wt%) minor components

### METHOD OF USE

The inventive product is used for suppressing appetite and/or controlling obesity in humans. Generally, at least one inventive product should be ingested per day, typically from 1 to 5 per day (per 24 hours), until reaching the desired weight, and then continuing with this regime to maintain the desired weight. Most preferably, from 1 to 3 products are consumed per day for optimum effect. Most preferably, the inventive products are consumed for at least 14 consecutive days.

While the above summarizes the present invention, it will become apparent to those skilled in the art that modifications, variations and alterations may be made without deviating from the scope and spirit of the present invention as described and claimed herein. The invention will now be further illustrated in the following non-limiting examples.

### EXAMPLE 1

Binary mixtures of lipids and emulsifiers were selected according to their miscibility, as determined by light microscopy. To determine the miscibility of lipids and emulsifiers, mixtures in ratios of lipid:emulsifier of 70:30, 40:60 and 10:90 were prepared. They were mixed and kept at 45°C for one hour. Subsequently, they were vortexed again for one minute and left to cool down to room temperature. One to two drops of each mixture were then put into a 96-well plate with a flat bottom. The samples were investigated under a light microscope connected to a digital camera and computer system using a magnification of 100. Samples which yielded a clear solution were selected for further study.

**TABLE 3**

| Lipid | Emulsifier | Mixing Ratio (Lipid: Emulsifier in %) | | |
|---|---|---|---|---|
| | | A (70:30) | B (40:60) | C (10:90) |
| Glyceryl Monolinoleate | Lauroyl macrogol glycerides | immiscible | immiscible | immiscible |
| | Stearoyl macrogolglycerides | immiscible | immiscible | immiscible |
| | Caprylocaproyl macrogolglycerides | clear solution | clear solution | clear solution |
| | Propylene glycol monolaurate | clear solution | clear solution | clear solution |
| | Propylene glycol monocaprylate | clear solution | clear solution | clear solution |
| | Polyglyceryl Oleate | clear solution | clear solution | clear solution |
| | Purified diethylene glycol monoethyl ether | clear solution | clear solution | clear solution |
| Glyceryl Monooleate | Lauroyl macrogol glycerides | immiscible | immiscible | immiscible |
| | Stearoyl macrogolglycerides | immiscible | immiscible | immiscible |
| | Caprylocaproyl macrogolglycerides | clear solution | clear solution | clear solution |
| | Propylene glycol monolaurate | clear solution | clear solution | clear solution |
| | Propylene glycol monocaprylate | clear solution | clear solution | clear solution |
| | Polyglyceryl Oleate | clear solution | clear solution | clear solution |
| | Purified diethylene glycol monoethyl ether | clear solution | clear solution | clear solution |

### EXAMPLE 2

Binary lipid/emulsifier mixtures were evaluated with respect to the physical stability of the formulations in absence and presence of Hoodia extract with time. They were mixed in scintillation vials with the aid of magnetic stirring and kept at 45°C for approximately one hour. Subsequently, they were vortexed for one minute and allowed to cool down to room temperature. The mixtures were visually observed for their physical stability upon preparation and after two months' storage at ambient conditions for clarity/ turbidity as well as color changes. The mixtures that were evaluated are identified in Table 4.

**TABLE 4**

| Lipid | Emulsifier | Mixing Ratio (Lipid: Emulsifier in %) | | |
|---|---|---|---|---|
| | | A (70:30) | B (40:60) | C (10:90) |
| Glyceryl Monolinoleate | 1. Propylene glycol monolaurate | 11A | 11B | 11C |
| | 2. Propylene glycol monocaprylate | 12A | 12B | 12C |
| | 3. Polyglyceryl oleate | 13A | 13B | 13C |
| Glyceryl Monolinoleate | 1. Propylene glycol monolaurate | 21A | 22B | 21C |
| | 2. Propylene glycol monocaprylate | 22A | 22B | 22C |
| | 3. Polyglyceryl oleate | 23A | 23B | 23C |

The results that were obtained are summarized in Tables 5 and 6.

**TABLE 5 Stability of lipid-emulsifier mixtures after two months' storage (without Hoodia extract)**

| **Code** | **Appearance** |
|---|---|
| 11A | Obvious precipitation, color changed to pale orange |
| 11 B | Moderate precipitation, color changed to pale orange |
| 11C | Stable, clear solution was observed. |
| 12A | Obvious precipitation, color changed to pale orange |
| 12B | Color changed to pale orange, no precipitation |
| 12C | Stable, clear solution was observed. |
| 13A | Obvious precipitation, color changed to pale orange |
| 13B | Moderate precipitation, color changed to pale orange |
| 13C | Stable, clear solution was observed. |
| 21A | Moderate precipitation |
| 21B | Stable, clear solution was observed. |
| 21C | Stable, clear solution was observed. |
| 22A | Moderate precipitation |
| 22B | Stable, clear solution was observed. |
| 22C | Stable, clear solution was observed. |
| 23A | Moderate precipitation |
| 23B | Stable, clear solution was observed. |
| 23C | Stable, clear solution was observed. |

The appearance of most of tested lipid-emulsifier mixtures did not change during two months' storage at ambient conditions except for some lipid-emulsifier mixtures containing Glyceryl Monolinoleate. These showed turbidity after two months' storage and all underwent color changes, slowly changing from transparent to pale orange.

### Compatibility of Lipid-Emulsifier Mixtures with Hoodia Extract

In addition to the pure lipid-emulsifier mixtures, the corresponding mixtures were prepared with the recommended dose of Hoodia extract (73% purity), i.e. 400 mg of the active (equivalent to 520 mg of the extract), per 10 g of vehicle mixture. The extract was dissolved by gradually adding into the vehicle mixture in a scintillation glass vial with continuous stirring at room temperature with the aid of magnetic bar. The appearance of the formulations immediately after incorporation of the extract was compared with their appearance after two months storage.

**TABLE 6 Stability of lipid-emulsifier mixtures after two months' storage (with Hoodia extract)**

| **Code** | **Appearance** |
|---|---|
| 11A | Turbid with high viscosity |
| 11B | Moderately turbid with some precipitate at the bottom, low viscosity |
| 11C | Slightly turbid with low viscosity |
| 12A | Turbid with medium viscosity |
| 12B | Clear solution with low viscosity |
| 12C | Clear solution with low viscosity |
| 13A | Turbid with very high viscosity |
| 13B | Moderately turbid with high viscosity |
| 13C | Slightly turbid with medium viscosity |
| 21A | Moderately turbid with medium viscosity |
| 21B | Moderately turbid with low viscosity |
| 21C | Slightly turbid with low viscosity |
| 22A | Moderately turbid with some precipitate at the bottom, low viscosity |
| 22B | Clear solution with low viscosity |
| 22C | Clear solution with low viscosity |
| 23A | Moderately turbid with very high viscosity |
| 23B | Slightly turbid with medium viscosity |
| 23C | Slightly turbid with medium viscosity |

Upon initial addition of Hoodia, the extract was completely dissolved in each of the lipid emulsifier mixtures, resulting in a dark green, clear solution. Some lipid-emulsifier mixtures developed turbidity over storage time.

### EXAMPLE 3

The following appetite suppressant Muesli Bar, Yoghurt Muesli Variant, is within the scope of the invention:

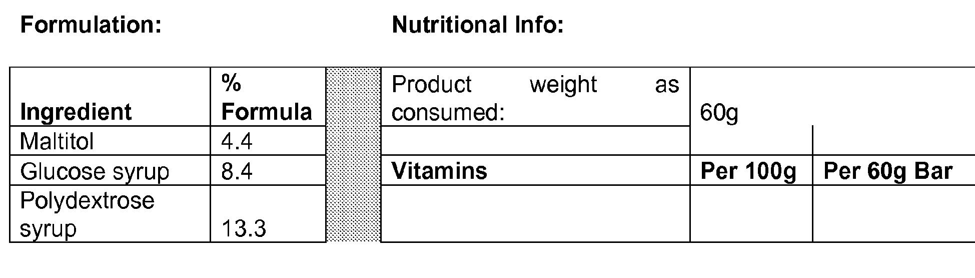

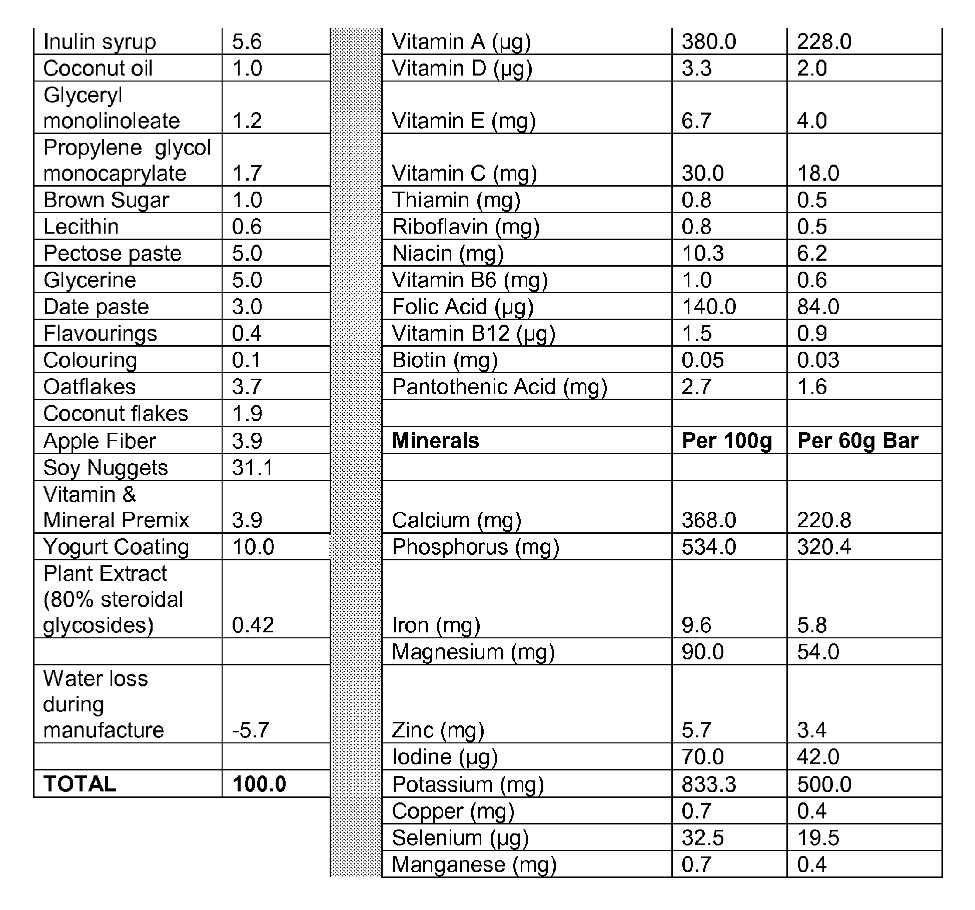

### EXAMPLE 4

The following appetite suppressant Chicken & Mushroom Soup is within a scope of the invention:

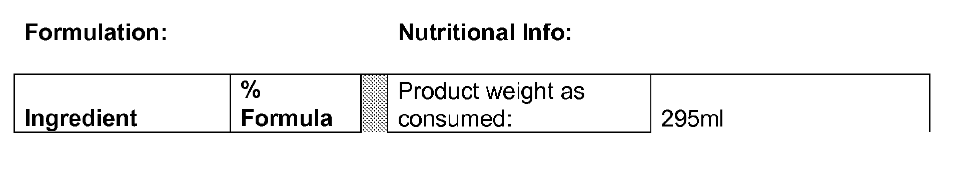

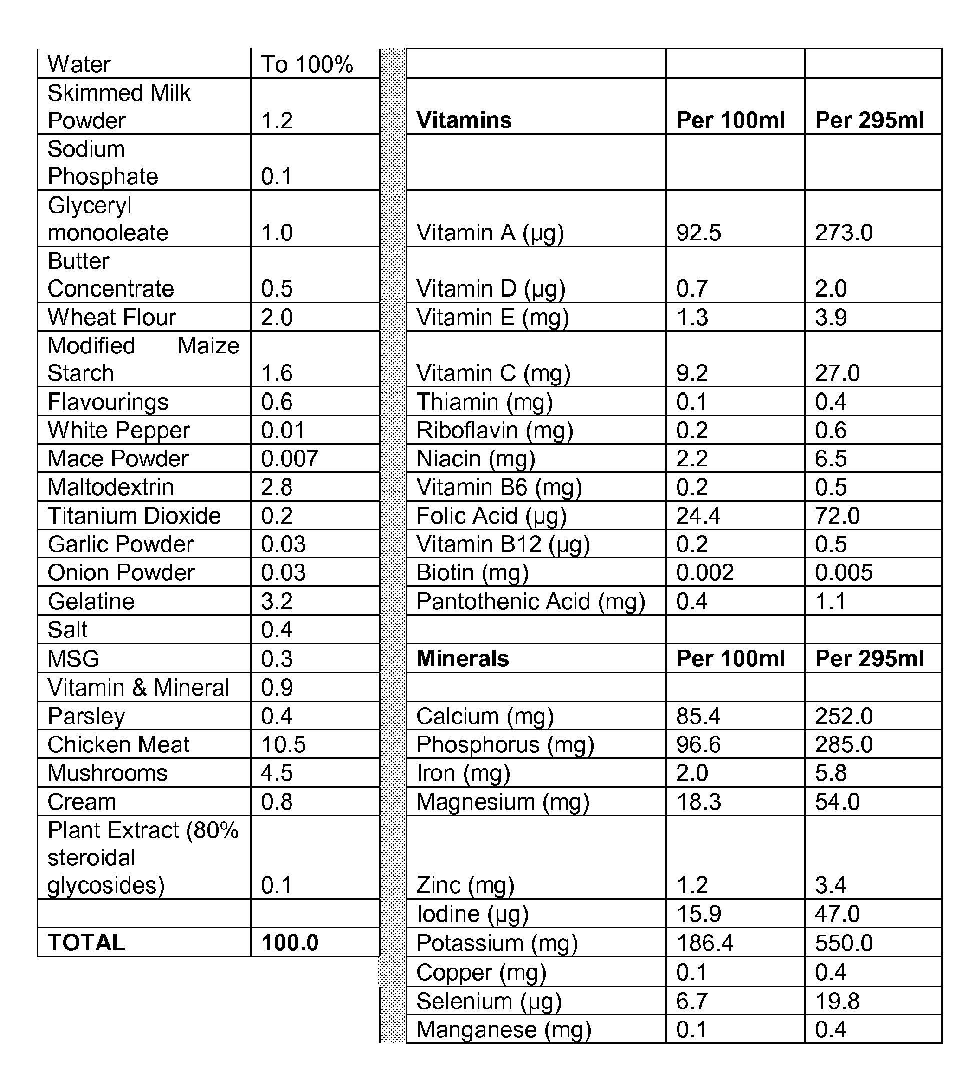

### EXAMPLE 5

The following appetite suppressant Strawberry Milk Drink is within the scope of the invention:

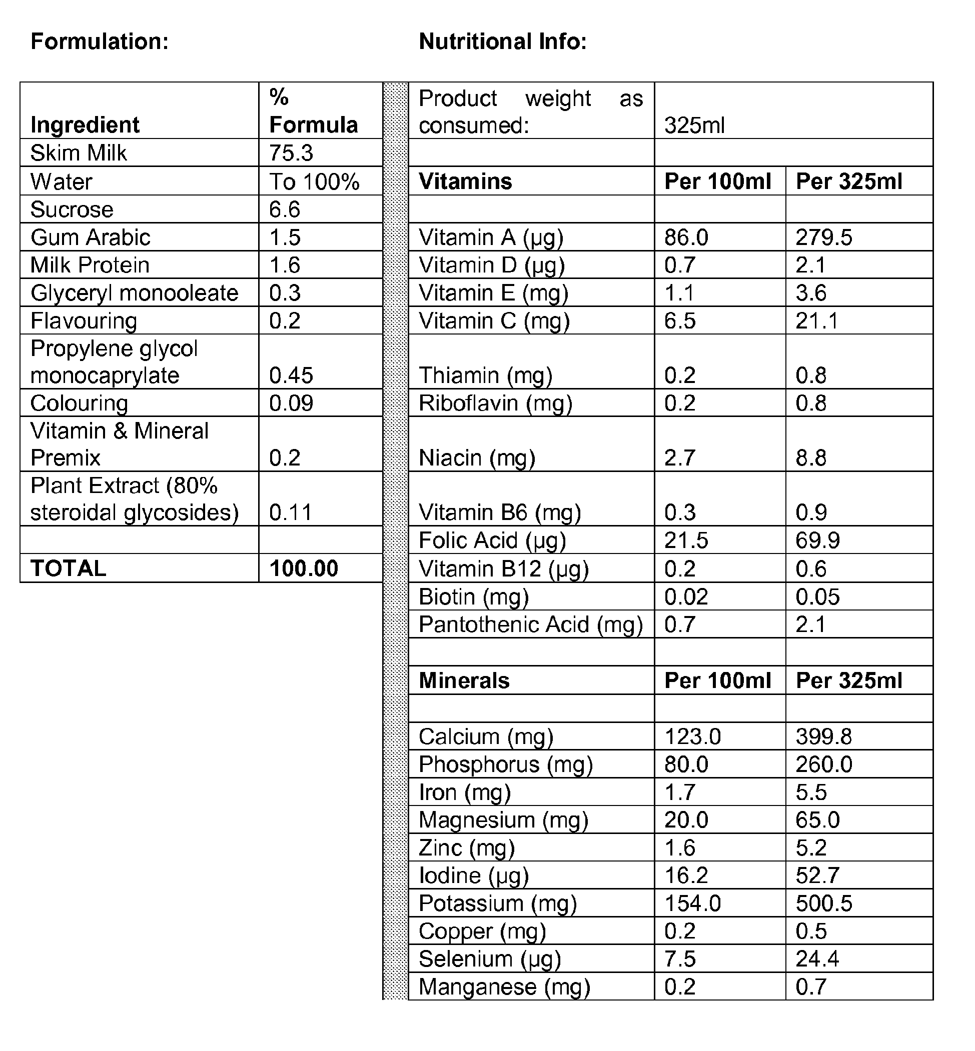

The 80% steroidal glycoside had the following specification:

| Specification item Physical description | Specification limit | Test method |
|---|---|---|
| Form | Free flowing powder, free from particulate contamination | In-house |
| Colour | Light Yellow-green | In-house |
| Active Components Steroidal | > 80% | HPLC |
| glycosides Water content | < 3% | Karl Fischer |
| Residual solvents | | |
| Heptane | <0.5% | |
| Ethanol | <0.5 % | |
| Particle size | < 50 microns | In-house |
| Microbiological examination | | Ph. Eur. |
| Total viable | | |
| aerobic count Aerobic | ≤ 10⁴ cfu/g | |
| bacteria | | |
| Fungi | ≤ 10² cfu/g | |
| Enterobacteriace ae | ≤ 10² cfu/g | |
| *Escherichia coli* | Absent in 1 g | |
| *Staphylococcus aureus* | Absent in 1 g | |
| *Salmonella* spp | Absent in 10 g | |

### EXAMPLE 6

The following appetite reducing acidified dairy drink was prepared:

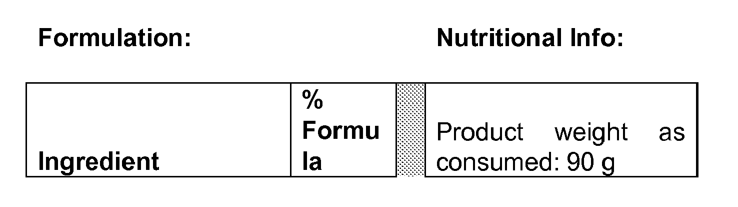

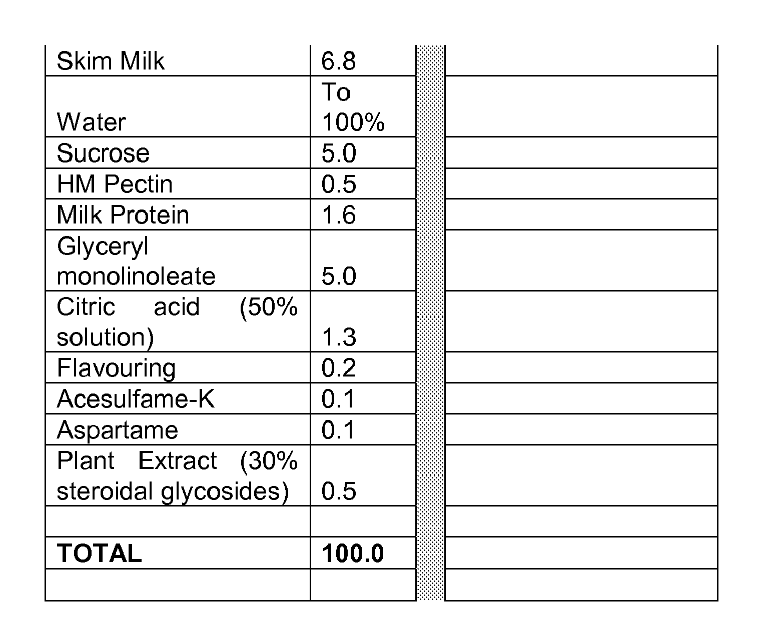

The 30% extract had the following specification:

| Specification item Physical description | Specification limit | Test method |
|---|---|---|
| Form | Powder, free from particulate contamination | In-house |
| Colour | Yellow-green | In-house |
| Active Components | | |
| Steroidal glycosides | > 30% | HPLC |
| Water content | < 5% | Karl Fischer |
| Residual solvents | | |
| Heptane | <1% | |
| Ethanol | <1 % | |
| Particle size | < 100 microns | In-house |
| Microbiological examination Total viable aerobic count | | Ph. Eur. |
| Aerobic bacteria | ≤ 10⁴ cfu/g | |
| Fungi | ≤ 10² cfu/g | |
| Enterobacteriaceae | ≤ 10² cfu/g | |
| *Escherichia coli* | Absent in 1 g | |
| *Staphylococcus aureus* | Absent in 1 g | |
| *Salmonella* spp | Absent in 10 g | |

While described in terms of the presently preferred embodiments, it is to be understood that such disclosure is not to be interpreted as limiting. Various modifications and alterations will no doubt occur to one skilled in the art after having read the above disclosure. Accordingly, it is intended that the appended claims be interpreted as covering all such modifications and alterations as falling within the true spirit and scope of the invention.

## Claims

1. An edible oil composition comprising:
(i) from about 10% to about 95%, by weight of the composition, of a food grade monoacylglyceride of an unsaturated fatty acid;
(ii) from about 1 % to about 90%, by weight of the composition, of Hoodia plant extract comprising steroidal glycosides.

2. The composition of claim 1 wherein the extract comprises from about 15% to about 90% of steroidal glycosides, by weight of the extract.

3. The composition of claim 2 wherein the extract comprises at least about 80% steroidal glycosides, by weight of the extract.

4. The composition of claim 1 wherein the unsaturated fatty acid comprises at least 12 carbon atoms.

5. The composition of claim 1 wherein the monoacylglyceride of an unsaturated fatty acid is selected from the group consisting mono-glyceryl oleate and glyceryl linoleate.

6. The composition of claim 1 further comprising from about 0.1 % to about 85%, by weight of the composition, of a food grade emulsifier.

7. The composition of claim 6 wherein the emulsifier is selected from the group consisting of propylene glycol carboxylic acid mono-ester, polyglyceryl oleate and diethylene glycol monoethyl ether.

8. The composition of claim 7 wherein the emulsifier is selected from the group consisting of propylene glycol monolaurate, propylene glycol monocaprylate and polyglyceryl oleate.

9. The composition of claim 6 wherein the weight ratio of the monoacylglyceride of unsaturated fatty acid to the emulsifier is in the range of from about 5:95 to about 95:5.

10. The composition of claim 1 wherein the composition has a viscosity in the range of from 0.001 Pa.s to 5000 Pa.s.

11. The emulsion of claim 1 wherein the composition is stable for at least 3 days at ambient temperature.

12. The composition of claim 1 wherein the Hoodia extract selected from the group consisting of *Trichocaulon piliferum, Trichocaulon officinale, Hoodia currorii, Hoodia gordonii, Hoodia lugardii* and mixtures thereof.

13. The composition of claim 1 wherein the plant extract is *Hoodia Gordonii* extract.

14. The composition of claim 1 wherein the steroidal glycosides comprise a compound of Formula (1): 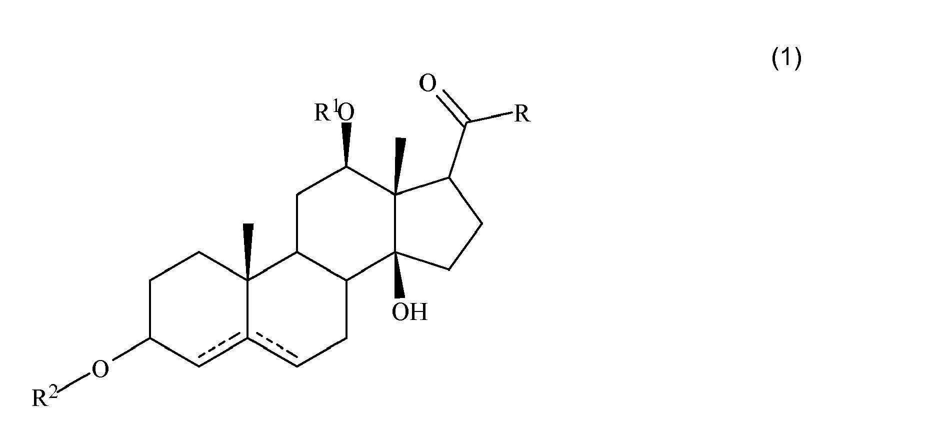 wherein
R = alkyl;
R¹ = H, alkyl, tigloyl, benzoyl or any other organic ester group, most preferably to optimize efficacy tigloyl;
R² = H or one or more 6-deoxy carbohydrates, or one or more 2,6-dideoxy carbohydrates, or glucose radical, or combinations thereof; and
wherein the broken lines indicate the optional presence of a further bond between carbon atoms C4 and C5 or between carbon atoms C5 and C6.

15. The composition of claim 1 wherein the oil phase is an isotropic clear solution.

16. The composition of claim 1, wherein the composition has a green color.

17. An edible appetite-suppressing oil-in-water emulsion comprising from about 0.1 % to about 90% of the oil composition according to claim 1.

18. The emulsion of claim 17 wherein the oil composition is in the form of dispersed green-colored droplets.

19. An edible appetite suppressant product comprising the emulsion of claim 17.

20. The product of claim 17 in the form of a drink.

21. An edible appetite-suppressing water-in-oil emulsion comprising from about 10% to about 99.9% of the oil composition according to claim 1.
